# FASCICULE DE BREVET EUROPEEN

(11) **EP 2 300 411 B1**
(45) Date de publication et mention de la délivrance du brevet: **12.02.2014**
(21) Numéro de dépôt: 09740358.8
(22) Date de dépôt: 22.07.2009
(51) Int. Cl.: C07C 45/52, C07C 51/377, C07C 69/15, C07C 69/24, C07C 67/055, C12P 7/06, C07C 1/24, C07C 11/04, C12P 7/54, C07C 51/235, C07C 57/04, C07C 51/36, C07C 53/122, C08F 18/08, C08F 18/10, C12P 7/62

(54) **PROCEDE DE FABRICATION D'ACIDE PROPIONIQUE BIO-RESSOURCE A PARTIR DE GLYCEROL**
VERFAHREN ZUR HERSTELLUNG VON AUS BIOLOGISCHEN QUELLEN STAMMENDER PROPIONSÄURE AUS GLYCERIN
METHOD FOR PRODUCING BIORESOURCED PROPIONIC ACID FROM GLYCEROL

(30) Priorité: 22.07.2008 FR 0854976
(43) Date de publication de la demande: 30.03.2011
(73) Titulaire: Arkema France, 92700 Colombes (FR)
(72) Inventeur: DUBOIS, Jean-Luc, F-69390 Millery (FR)
(74) Mandataire: Gavin, Pablo
(86) Numéro de dépôt international: PCT/FR2009/051470
(87) Numéro de publication internationale: WO 2010/010298

(56) Documents cités:
- EP-A- 1 710 227
- WO-A1-03/095411
- FR-A1- 2 909 999
- E. H. HIMMI ET AL: "Propionic acid fermentation of glycerol and glucose by Propionibacterium acidipropionici and Propionibacterium freudenreichii ssp. shermanii", APPLIED MICROBIOLOGY AND BIOTECHNOLOGY, vol. 53, no. 4, 12 avril 2000 (2000-04-12) , pages 435-440, XP55000653, ISSN: 0175-7598, DOI: 10.1007/s002530051638
- F. BARBIRATO ET AL: "Propionic acid fermentation from glycerol: comparison with conventional substrates", APPLIED MICROBIOLOGY AND BIOTECHNOLOGY, vol. 47, no. 4, 14 avril 1997 (1997-04-14) , pages 441-446, XP55000655, ISSN: 0175-7598, DOI: 10.1007/s002530050953
- JEFFERSON CORAL ET AL: "Batch Fermentation Model of Propionic Acid Production by Propionibacterium acidipropionici in Different Carbon Sources", APPLIED BIOCHEMISTRY AND BIOTECHNOLOGY, vol. 151, no. 2-3, 2 avril 2008 (2008-04-02), pages 333-341, XP55000656, ISSN: 0273-2289, DOI: 10.1007/s12010-008-8196-1

## Description

La présente invention vise un procédé de fabrication d'un acide propionique bio-ressourcé à partir de glycérol comme matière première, le terme acide bio-ressourcé indiquant que l'acide propionique est essentiellement obtenu à partir sur une source de carbone d'origine renouvelable.

L'acide propionique est une matière qui peut être utilisée comme solvant, comme conservateur alimentaire ou pour la fabrication d'herbicide ; l'acide propionique entre aussi dans la préparation du propionate de vinyle qui est utilisé comme monomère dans des (co)polymères avec par exemple l'éthylène, le chlorure de vinyle, ou les esters (meth)acryliques.

Des procédés de synthèse de l'acide propionique sont connus de l'art antérieur. Par exemple, la demande de brevet DE 102 25 339 A1 décrit un procédé de préparation de l'acide propionique par hydrogénation catalytique d'acide acrylique en présence d'oxygène moléculaire, d'un catalyseur d'un élément du groupe 8 à 11. Classiquement, l'acide acrylique est obtenu par oxydation catalytique en phase gazeuse de propane, propylène et/ou acroléine.

Un des problèmes posés par les procédés de synthèse de l'acide propionique de l'art antérieur est qu'ils sont réalisés à partir de matières premières d'origine fossile (pétrole) non renouvelables notamment le propane ou le propylène. Or les ressources en ces matières premières sont limitées, l'extraction du pétrole requiert d'aller creuser de plus en plus profond et dans des conditions techniques toujours plus difficiles nécessitant des équipements sophistiqués et la mise en oeuvre de procédés toujours plus coûteux en énergie. Ces contraintes ont une conséquence directe sur le coût de fabrication de l'acide propionique.

Par ailleurs, les industriels ont orienté leurs travaux de recherche et de développement depuis quelques années sur des procédés de synthèse dits « bio-ressourcés » utilisant des matières premières naturelles renouvelables.

Par exemple, pour la fabrication d'acide acrylique issu de ressources renouvelables, des procédés alternatifs à partir de matières premières végétales non fossiles ont été développés récemment. En particulier, des procédés à partir de glycérol (appelé aussi glycérine), issu de la méthanolyse des corps gras ont été développés. Ce glycérol est disponible en grande quantité et peut être stocké et transporté sans difficulté.

La méthanolyse des huiles végétales ou des graisses animales peut s'effectuer selon différents procédés bien connus , notamment en utilisant une catalyse homogène telle que la soude ou le méthylate de sodium en solution dans le méthanol, ou en utilisant une catalyse hétérogène. On pourra se reporter sur ce sujet à l'article de D. Ballerini et al. dans l'Actualité Chimique de nov-déc 2002.

En ce qui concerne la transformation du glycérol par voie chimique, on peut citer la synthèse de l'acide acrylique en deux étapes à savoir l'obtention de l'acroléine par déshydratation de glycérol, qui est notamment décrite dans le brevet US 5,387,720, suivie d'une oxydation « classique » de l'acroléine pour obtenir l'acide acrylique.

La première étape de la fabrication d'acide acrylique à partir de glycérol conduit au même composé intermédiaire que le procédé de fabrication conventionnel à partir de propylène, à savoir l'acroléine selon la réaction :

CH₂OH-CHOH-CH₂OH → CH₂=CH-CHO + 2H₂O

qui est suivie de la deuxième étape d'oxydation selon la réaction

CH₂=CH-CHO +½ O₂ → CH₂=CH-COOH

Les demandes de brevet EP 1.710.227, WO2006/136336 et WO2006/092272 décrivent de tels procédés de synthèse d'acide acrylique à partir de glycérol comportant l'étape de déshydratation en phase gaz en présence de catalyseurs constitués par des oxydes minéraux (mixtes ou non) à base d'aluminium, titane, zirconium, vanadium ... et l'étape d'oxydation en phase gaz de l'acroléine ainsi synthétisé en présence de catalyseurs à base d'oxydes de fer, molybdène, cuivre...seuls ou en combinaison sous forme d'oxydes mixtes.

Cependant, un des problèmes posés par ces procédés est que l'acide acrylique n'est pas le seul produit formé et que des sous-produits sont formés en quantité importante, tels que l'acide propionique et des impuretés comme l'eau, les dimères d'acide acrylique, l'acide acétique, l'acroléine, le benzaldéhyde, les furfurals ou encore l'hydroquinone. Il est donc généralement nécessaire de purifier l'acide acrylique par les techniques classiques afin d'obtenir une solution d'acide acrylique plus concentrée.

La qualité de l'acide acrylique c'est-à-dire sa teneur en différentes impuretés jouant un grand rôle dans les processus de polymérisation ultérieurs les industriels fabriquant cet acide acrylique ont été conduits à mettre en jeu toute une série d'étapes de purification afin d'obtenir un acide acrylique « standard » qu'on appelle habituellement l'acide acrylique glacial (AAg). Cet AAg ne répond pas à des spécifications reconnues officiellement et ayant un caractère universel mais signifie pour chaque industriel le niveau de pureté à atteindre pour pouvoir conduire avec succès ses transformations ultérieures. A titre d'exemple pour un acide acrylique ex propylène, l'effluent de sortie de réacteur est soumis à une combinaison d'étapes qui peuvent différer par leur enchaînement selon le procédé : élimination des incondensables et de l'essentiel des composés très légers, en particulier l'acroléine intermédiaire de synthèse de l'acide acrylique (AA brut), déshydratation éliminant l'eau et le formol (AA déshydraté), élimination des légers (en particulier l'acide acétique), élimination des lourds, éventuellement élimination de certaines impuretés résiduelles par traitement chimique. Ce procédé présente avec le procédé de synthèse à partir du propylène une grande analogie dans la mesure où le produit intermédiaire, l'acroléine, issu de la première étape est le même et que la seconde étape est conduite dans les mêmes conditions opératoires. Cependant la réaction de première étape du procédé de l'invention, réaction de déshydratation, est différente de la réaction d'oxydation du propylène du procédé habituel. La réaction de déshydratation menée en phase gaz est conduite au moyen de catalyseurs solides différents de ceux utilisés pour l'oxydation du propylène. L'effluent riche en acroléine issu de la première étape de déshydratation, destiné à alimenter la seconde étape d'oxydation de l'acroléine en acide acrylique, contient une quantité plus importante d'eau, et présente en outre des différences sensibles en matière de sous-produits découlant des mécanismes réactionnels mis en jeu se concrétisant par des sélectivités différentes dans chacune des deux voies.

Pour illustrer ces différences le tableau 1 ci-dessous rassemble les données concernant la présence de divers acides dans l'acide acrylique brut c'est-à-dire dans la phase liquide sortant du réacteur de seconde étape selon l'état de l'art actuel.

**Tableau 1**

| **Ratio massique impureté / AA (acide acrylique brut)** | Procédé ex propylène | Procédé ex glycérol |
|---|---|---|
| Acide acétique / AA | < 5% | > 10% |
| Acide propionique / AA | < 0.1% | > 0.5% |

Le tableau 1 illustre certaines des principales différences, en terme de constituants de l'effluent liquide sortant du réacteur d'oxydation, entre les procédés ex-propylène et ex-glycérol. Naturellement, bien que cela ne soit pas mentionné dans le tableau, on trouve également dans l'acide acrylique brut, qu'il provienne du procédé ex-propylène ou du procédé ex-glycérol toute une série de composés oxygénés, alcools, aldéhydes, cétones, autres acides dont la séparation, nécessaire, est connue de l'homme de l'art.

L'acide acétique et l'acide propionique sont gênants pour l'acide acrylique en particulier parce qu'il ne sont pas transformés lors du processus de polymérisation , ils sont saturés et donc non polymérisables ; selon le procédé de polymérisation mis en jeu et les applications visées pour le polymère, ces impuretés peuvent rester dans le produit fini et risquer de conférer au produit fini des propriétés corrosives indésirables, ou se retrouver dans les rejets liquides ou gazeux générés par le procédé de polymérisation, et occasionner une pollution organique également non souhaitable. Ils doivent donc être éliminés.

L'élimination de l'acide acétique peut être obtenue par distillation dans une fraction légère, opération généralement désignée par étêtage. Toutefois, la réduction de la concentration d'acide acétique dans le cadre du procédé ex-glycérol entraîne une perte conséquente d'acide acrylique dans la fraction légère, du fait d'une part de l'écart important existant entre sa teneur initiale dans l'acide acrylique brut et sa teneur visée dans l'acide acrylique technique et d'autre part de l'existence de liaisons hydrogène existant entre les groupements carboxyliques des deux molécules. Cet inconvénient est important sur le plan économique car l'obtention d'un acide acrylique glacial à teneur en acide acétique inférieure à 0,1 % poids ne peut se faire qu'au détriment du taux de récupération de l'acide acrylique sortant du réacteur d'oxydation.

Pour ce qui concerne l'acide propionique, la différence de volatilité extrêmement faible existant entre cette impureté à éliminer et l'acide acrylique à purifier (de l'ordre de 1°C) empêche toute purification de l'acide acrylique dans des conditions économiquement acceptables par distillation.

Il n'existe dans l'art antérieur aucun procédé permettant la fabrication de compositions suffisamment concentrées en acide propionique d'origine renouvelable pour permettre leur utilisation dans les applications classiques de l'acide propionique obtenu par des matières premières fossiles.

De manière avantageuse et surprenante, la demanderesse de la présente demande a mis en oeuvre un procédé de fabrication industriel de l'acide propionique à partir de glycérol.

Le procédé selon l'invention permet de s'affranchir au moins en partie des matières premières d'origine fossile et de les remplacer par des matières premières renouvelables.

L'acide propionique obtenu suivant le procédé selon l'invention est de qualité tel qu'il peut être utilisé dans toutes les applications dans lesquelles il est connu d'utiliser l'acide propionique, y compris dans les applications les plus exigeantes.

L'invention a pour objet un procédé de fabrication d'acide propionique bio-ressourcé à partir de glycérol comportant les étapes suivantes :
- déshydratation catalytique en phase gaz du glycérol en acroléine, (1)
- condensation partielle par refroidissement et extraction d'une partie de l'eau contenue dans le milieu réactionnel de (1), (1')
- oxydation catalytique en phase gaz de l'acroléine en acide acrylique, (2)
- extraction de l'acide acrylique contenu dans l'effluent de l'oxydation par absorption avec un solvant, (3)
- séchage de la solution d'acide acrylique par distillation en présence d'un solvant non miscible à l'eau, (4)
- distillation de la solution ainsi obtenue pour éliminer les composés légers (étêtage), (5)
- distillation de la fraction lourde issue de l'étape précédente (5) pour éliminer les composés lourds (équeutage), (6)
   combinées avec une étape d'extraction de l'acide acrylique par cristallisation fractionnée appliquée à l'un des effluents suivants : la fraction lourde de (4), fraction lourde de (5) ou fraction légère de (6) pour isoler des cristaux d'acide acrylique purifié et une solution d'eaux-mères appauvrie en acide acrylique.
- hydrogénation catalytique des eaux mères isolées à l'étape de cristallisation fractionnée en présence d'hydrogène moléculaire pour former une solution d'acide propionique
- séparation de l'acide propionique, par exemple par distillation.

Le procédé selon l'invention permet d'obtenir un acide propionique bio-ressourcé obtenu à partir de ressources renouvelables.

Une matière première renouvelable est une ressource naturelle dont le stock peut se reconstituer sur une période courte à l'échelle humaine. Il faut en particulier que ce stock puisse se renouveler aussi vite qu'il est consommé. Par exemple, les matières végétales présentent l'avantage de pouvoir être cultivées sans que leur consommation aboutisse à une diminution apparente des ressources naturelles.

A la différence des matériaux issus de matières fossiles, les matières premières renouvelables contiennent du ¹⁴C . Tous les échantillons de carbone tirés d'organismes vivants (animaux ou végétaux) sont en fait un mélange de 3 isotopes : ¹²C (représentant environ 98,892 %), ¹³C (environ 1,108 %) et ¹⁴C (traces: 1,2.10⁻¹⁰ %). Le rapport ¹⁴C/¹²C des tissus vivants est identique à celui de l'atmosphère. Dans l'environnement, le ¹⁴C existe sous deux formes prépondérantes : sous forme de gaz carbonique (CO₂), et sous forme organique, c'est-à-dire de carbone intégré dans des molécules organiques.

Dans un organisme vivant, le rapport ¹⁴C/¹²C est maintenu constant par le métabolisme car le carbone est continuellement échangé avec l'environnement extérieur. La proportion de ¹⁴C étant constante dans l'atmosphère, il en est de même dans l'organisme, tant qu'il est vivant, puisqu'il absorbe ce ¹⁴C au même titre que le ¹²C ambiant. Le rapport moyen de ¹⁴C/¹²C est égal à 1,2x10⁻¹².

Le ¹²C est stable, c'est-à-dire que le nombre d'atomes de ¹²C dans un échantillon donné est constant au cours du temps. Le ¹⁴C est radioactif, le nombre d'atomes de ¹⁴C dans un échantillon décroît au cours du temps (t), sa demi-vie étant égale à 5730 ans.

La teneur en ¹⁴C est sensiblement constante depuis l'extraction des matières premières renouvelables, jusqu'à la fabrication de l'acide propionique bio-ressourcé et même jusqu'à la fin de l'utilisation de l'objet comprenant de l'acide propionique.

Par conséquent, la présence de ¹⁴C dans un matériau, et ce, quelle qu'en soit la quantité, donne une indication sur l'origine des molécules le constituant, à savoir qu'elles proviennent de matières premières renouvelables et non de matériaux fossiles.

La quantité de ¹⁴C dans un matériau peut être déterminée par l'une des méthodes décrites dans la norme ASTM D6866-06 (Standard Test Methods for Determining the Biobased Content of Natural Range Materials Using Radiocarbon and Isotope Ratio Mass Spectrometry Analysis).

Cette norme comporte trois méthodes de mesure du carbone organique issu de matières premières renouvelables, dénommé en langue anglaise « biobased carbon ». Les proportions indiquées pour l'acide propionique de l'invention sont de préférence mesurées selon la méthode par spectrométrie de masse ou la méthode par spectrométrie à scintillation liquide décrite dans cette norme, et tout préférentiellement par spectrométrie de masse.

Ces méthodes de mesure évaluent le rapport des isotopes ¹⁴C/¹²C dans l'échantillon et le comparent à un rapport des isotopes ¹⁴C/¹²C dans un matériau d'origine biologique donnant le 100% standard, afin de mesurer le pourcentage de carbone organique de l'échantillon.

De préférence, l'acide propionique selon l'invention comprend une quantité de carbone issu de matières premières renouvelables supérieure à 20%, de préférence supérieure à 40% en masse par rapport à la masse totale de carbone de l'acide propionique.

En d'autres termes, l'acide propionique peut comporter au moins 0,25 10⁻¹⁰ % en masse de ¹⁴C, et de préférence au moins 0,5 10⁻¹⁰ % en masse¹⁴C.

Avantageusement la quantité de carbone issu de matières premières renouvelables est supérieure à 75%, de préférence égale à 100% en masse par rapport à la masse totale de carbone de l'acide propionique.

Selon le procédé de l'invention, on purifie l'acide acrylique par cristallisation fractionnée. Lors de cette purification, on obtient deux flux : un premier flux concentré en acide acrylique qui pourra être valorisé en tant qu'acide acrylique et un second flux (eaux-mères) plus pauvre en acide acrylique. Ce second flux ne peut être valorisé en tant qu'acide acrylique. Selon le procédé de l'invention, ce second flux est hydrogéné pour former de l'acide propionique bio-ressourcé. Le procédé de fabrication selon l'invention permet d'obtenir un acide acrylique purifié ainsi que de valoriser le second flux et limiter ainsi les pertes de produit lors du procédé.

Pour la mise en oeuvre du procédé on utilise généralement un flux alimentant le réacteur de l'étape (1) contenant le glycérol et de l'eau, avec un ratio massique eau / glycérol pouvant varier dans de larges mesures, par exemple entre 0,04 / 1 et 9 / 1 et de préférence entre 0,7 / 1 et 3 / 1. La réaction de déshydratation, étape (1), qui est une réaction équilibrée mais favorisée par un niveau de température élevée, est effectuée en général en phase gaz dans le réacteur en présence d'un catalyseur à une température allant de 150°C à 500°C, de préférence comprise entre 250°C et 350°C, et une pression absolue comprise entre 1 et 5 bars (1 000 et 5 000kPa). On peut aussi l'effectuer en présence d'oxygène ou d'un gaz contenant de l'oxygène comme décrit dans les demandes WO 06/087083 et WO 06/114506.

La réaction de déshydratation du glycérol est généralement effectuée, sur des catalyseurs solides acides. Les catalyseurs qui conviennent sont des matériaux utilisés dans un milieu réactionnel gazeux ou liquide, en phase hétérogène, qui ont une acidité de Hammett, notée H₀ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse.

Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides.

Ces catalyseurs pourront généralement être constitués par un sel d'hétéropolyacide dans lequel des protons dudit hétéropolyacide sont échangés avec au moins un cation choisi parmi les éléments appartenant aux Groupes 1 à XVI de la Classification Périodique des Eléments, ces sels d'hétéropolyacide contenant au moins un élément choisi parmi le groupe comprenant W, Mo et V.

Parmi les oxydes mixtes, on peut citer particulièrement ceux à base de fer et de phosphore et ceux à base de césium, phosphore et tungstène.

Les catalyseurs sont notamment choisis parmi les zéolithes, les composites Nafion® (à base d'acide sulfonique de polymères fluorés), les alumines chlorées, les acides et sels d'acides phosphotungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₃, imprégnés de fonctions acides telles que borate BO₃, sulfate SO₄, tungstate WO₃, phosphate PO₄, silicate SiO₂, ou molybdate MoO₃' ou un mélange de ces composés.

Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni, or montmorillonite.

Les catalyseurs préférés sont les zircones phosphatées, les zircones tungstées, les zircones silicées, les oxydes de titane ou d'étain imprégnés de tungstate ou phosphotungstate, les alumines ou silices phosphatées, les hétéropolyacides ou sels d'hétéropolyacides, les phosphates de fer et les phosphates de fer comprenant un promoteur.

Le milieu réactionnel sortant du réacteur de déshydratation a une teneur en eau importante due à la charge de glycérol (solution aqueuse) et à la réaction elle-même. Une étape supplémentaire (1') de condensation partielle de l'eau telle que par exemple celle décrite dans la demande de brevet WO 08/087315 au nom de la déposante, permettra d'en éliminer une partie, de façon à amener ce gaz à une composition sensiblement identique à celle du procédé ex propylène, pour alimenter la deuxième étape d'oxydation de l'acroléine en acide acrylique. Par composition sensiblement identique, on entend en particulier des concentrations en acroléine, eau et oxygène proches. Cette étape de condensation (1') doit être conduite avec un refroidissement à une température permettant d'obtenir, après élimination de la phase condensée, un flux gazeux contenant de l'eau et de l'acroléine dans un ratio molaire eau / acroléine de 1,5 / 1 à 7 / 1 Cette condensation partielle de l'eau permet d'éviter une dégradation du catalyseur de 2^{ème} étage d'oxydation de l'acroléine en acide acrylique et d'éviter lors des étapes ultérieures l'élimination de grandes quantités d'eau qui est coûteuse et qui risque d'entraîner des pertes en acide acrylique. En outre elle permet d'éliminer une partie des impuretés « lourdes » formées lors de la déshydratation

La réaction d'oxydation, étape (2) s'effectue en présence d'oxygène moléculaire ou d'un mélange contenant de l'oxygène moléculaire, à une température allant de 200°C à 350°C, de préférence de 250°C à 320°C, et sous une pression allant de 1 à 5 bars en présence d'un catalyseur d'oxydation.

Comme catalyseur d'oxydation, on utilise tous types de catalyseurs bien connus de l'homme de l'art pour cette réaction. Généralement sont utilisés des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. En particulier, sont utilisées les formulations contenant Mo et/ou V et/ou W et/ou Cu et/ou Sb et/ou Fe comme constituants principaux.

Le mélange gazeux issu de l'étape (2) est constitué, en dehors de l'acide acrylique :
- de composés légers incondensables dans les conditions de températures et de pressions habituellement mises en oeuvre : azote, oxygène non converti, monoxyde et dioxyde de carbone formés en faible quantité par oxydation ultime,
- de composés légers condensables : en particulier l'eau, générée par la réaction de déshydratation ou présente comme diluant, l'acroléine non convertie, des aldéhydes légers, comme le formaldéhyde et l'acétaldéhyde, l'acide formique, l'acide acétique et l'acide propionique
- de composés lourds : furfuraldéhyde, benzaldéhyde, acide et anhydride maléique, acide 2-butènoïque, acide benzoïque, phénol, protoanémonine...

L'étape (3) consiste en une extraction de l'acide acrylique par absorption dans un solvant. Le solvant peut être de l'eau ou un mélange de solvants lourds hydrophobes comme le diphényle et le diphényl ether. Cette étape d'extraction est connue de l'homme du métier et celui-ci peut se référer aux brevets suivants : brevet français n° 1 588 432, brevet français n° 2 146 386, brevet allemand n° 4 308 087, brevet européen n° 0 706 986 et brevet français n° 2 756 280. On peut réaliser cette extraction avec de l'eau par une absorption à contre-courant. Pour cela, on introduit le gaz issu du réacteur en pied d'une colonne d'absorption où il rencontre à contre-courant de l'eau introduite en tête de colonne. Les composés légers (principalement acétaldéhyde et acroléine) sont pour l'essentiel éliminés en tête de cette colonne d'absorption. L'eau utilisée comme solvant absorbant peut être amenée par une source extérieure au procédé, mais sera constituée préférentiellement pour partie ou totalement par récupération à partir d'au moins un des flux gazeux réactionnels issus des étapes réactionnelles initiales par exemple l'eau issue des étapes (1') et (4), à savoir l'eau condensée dans l'étape 1', ou l'eau récupérée à partir du flux de tête de colonne de séchage azéotropique. Les conditions opérationnelles de cette étape d'absorption sont les suivantes :
Le mélange réactionnel gazeux est introduit en pied de colonne à une température comprise entre 130°C et 250°C. L'eau est introduite en tête de colonne à une température comprise entre 10°C et 60°C. Les quantités respectives d'eau et de mélange réactionnel gazeux sont telles que le ratio massique eau/ acide acrylique est compris entre 1/1et 1/4. L'opération est conduite à la pression atmosphérique.

Dans une variante préférée de mise en oeuvre du procédé, lors d'une étape (3') on procède à la récupération de l'acroléine, contenue dans la fraction liquide issue de (3), par distillation ou stripping avec un gaz. Dans cette variante du procédé la colonne d'absorption peut être couplée avec une colonne de distillation des composés très légers, essentiellement l'acroléine non convertie à l'issue de la réaction, présente en faible concentration dans la solution aqueuse d'acide acrylique récupérée en pied de colonne d'absorption. Cette colonne de distillation, fonctionnant sous une pression de 6.10³ à 7.10⁴ Pa, est alimentée en tête par le flux de pied de colonne d'absorption précédente, et permet d'éliminer en tête un flux d'acide acrylique enrichi en acroléine, qui est recyclé en partie inférieure de la colonne d'absorption (3), pour une élimination finale en tête de cette même colonne. On obtient ainsi un mélange aqueux d'acide acrylique dans l'eau (ratio massique 1/1 à 4/1) débarrassé de l'essentiel de l'acroléine non convertie, que l'on nomme "acide acrylique brut". La récupération de l'acroléine peut également être réalisée par stripping avec un gaz tel que l'air ou un mélange de gaz inerte contenant préférentiellement de l'oxygène

Cette étape est facultative mais en son absence l'acide acrylique brut sera plus concentré en acroléine qui devra être éliminée lors de l'étape ultérieure d'étêtage. Par ailleurs, cette étape (3') permet de récupérer et recycler l'acroléine à la section réaction (2) et ainsi d'augmenter le rendement global du procédé.

L'étape (4) est une étape de déshydratation ou séchage qui est réalisée en présence d'un solvant de l'acide acrylique non miscible à l'eau. Cette étape de déshydratation peut être réalisée par extraction liquide - liquide de l'acide acrylique en présence du solvant, suivie par une étape de séparation du monomère, acide acrylique, par distillation.

Cette phase de déshydratation est décrite dans de nombreux brevets, voir par exemple le brevet FR 2.119.764, avec la méthylisobutylcétone (MIBK) comme solvant, ou le brevet US 3,689,541, avec la triméthylcyclohexanone comme solvant, ou par distillation en présence de solvant ou de mélanges de solvants formant un azéotrope hétérogène avec l'eau, comme les acétates ou la méthylisobutylcétone tel que décrite par exemple dans le brevet FR 2.554.809 ou encore des solvants formant en outre un mélange azéotropique avec l'acide acétique comme le toluène tel que décrit par exemple dans le brevet JP 03.181.440.

Dans le procédé de l'invention, on utilisera de préférence pour cette étape de déshydratation une distillation azéotropique à l'aide d'un solvant tel que MIBK. La colonne de distillation qui fonctionne sous une pression de 6.10³ à 7.10⁴ Pa, est équipée d'un décanteur qui reçoit le flux de tête de colonne après condensation et assure la séparation d'une phase organique supérieure essentiellement constituée de MIBK, totalement recyclée en reflux en tête de colonne, et d'une phase aqueuse contenant l'eau et la majeure partie du formaldéhyde. La puissance de chauffe imposée au bouilleur de la colonne est régulée de façon à obtenir un débit de reflux de solvant tel que le ratio massique de solvant renvoyé en reflux et de l'eau contenue dans l'acide acrylique brut alimentant la colonne corresponde au mélange azéotropique théorique. Le flux obtenu en pied de colonne, l'acide acrylique déshydraté, est essentiellement exempt d'eau (généralement moins de 1% poids).

Dans une variante de réalisation cette colonne peut être couplée à une deuxième colonne de récupération du solvant, de façon à récupérer dans le flux aqueux décanté en tête de colonne de distillation azéotropique les traces de solvant solubilisées dans la phase aqueuse. Ces faibles quantités de solvant distillées et condensées en tête de cette colonne de récupération de solvant, fonctionnant sous pression atmosphérique, sont ensuite recyclées dans le décanteur de la colonne précédente. Le flux aqueux de pied de cette colonne de récupération de solvant est éliminé.

L'étape (5) est une étape d'élimination des composés légers, en particulier l'acide acétique et l'acide formique, par distillation ; elle est généralement dénommée « étêtage ». Le flux d'acide acrylique déshydraté obtenu en pied de la colonne de distillation azéotropique est envoyé en partie médiane d'une colonne à distiller qui fonctionne sous une pression de tête de l'ordre de 2.10³ à 2.10⁴ Pa. Le flux de pied de colonne contient l'acide acrylique débarrassé de l'essentiel des composés légers. Le flux de tête de colonne, riche en acide acétique et acide formique, peut éventuellement être traité de façon complémentaire pour récupérer dans une deuxième colonne en série les faibles quantités d'acide acrylique entraînées avec le flux de tête de colonne.

L'étape (6) est une étape de séparation des composés lourds par distillation. Le flux de pied de la colonne précédente d'étêtage est introduit en pied d'une colonne de distillation fonctionnant sous une pression de tête de l'ordre de 2.10³ à 2.10⁴ Pa. On obtient en tête un flux d'acide acrylique purifié, dit de grade technique.

Les différentes étapes de séparation par distillation imposent en raison des conditions thermodynamiques mises en oeuvre d'ajouter aux flux traités des inhibiteurs de polymérisation afin d'éviter la formation de composés lourds formés par polymérisation de l'acide acrylique, qui sont préjudiciables au bon fonctionnement de l'ensemble. Les inhibiteurs de polymérisation généralement utilisés pour les étapes de purification de l'acide acrylique sont les produits phénoliques, comme l'hydroquinone ou l'éther méthylique de l'hydroquinone, les dérivés de la phénothiazine, des composés de la famille des thiocarbamates, comme le di n-butyl dithiocarbamate de cuivre, des dérivés aminés, comme les hydroxylamines, l'hydroxydiphénylamine, ou de la famille des phénylène diamines, des dérivés nitroxydes de la 4-hydroxy 2,2,6,6-tetraméthyl-1-oxyl-pipéridine (TEMPO), comme le 4-hydroxy-TEMPO ou le 4-oxo-TEMPO, ou encore des sels métalliques, comme l'acétate de manganèse. Ces inhibiteurs peuvent être utilisés seuls ou en combinaison, et sont en outre préférentiellement introduits en association avec un gaz contenant de l'oxygène.

Ces inhibiteurs de polymérisation sont généralement des composés lourds, dont la volatilité est plus faible que celle de l'acide acrylique. Ils sont éliminés en fond des colonnes. En revanche, leur concentration dans la phase vapeur à l'intérieur des colonnes de distillation est faible et insuffisante pour éviter l'initiation de polymères. Pour éviter l'apparition et l'accumulation de polymères, ces additifs sont habituellement introduits dans les flux liquides alimentant les équipements, mais aussi en tête et en différents points des colonnes et équipements, de façon à assurer un reflux constant et homogène de solution riche en inhibiteurs de polymérisation sur toutes les parties des équipements. En général, ils sont envoyés en solution dans un liquide, par exemple dans l'acide acrylique ou dans l'eau si l'étape de purification concerne des flux aqueux.

Le procédé de l'invention comprend une étape de purification de l'acide acrylique bio-ressourcé qui est une séparation par cristallisation fractionnée.

La cristallisation fractionnée est une technique de séparation bien connue. Elle peut être mise en oeuvre sous différentes formes, cristallisation dynamique, cristallisation statique ou cristallisation en suspension. On peut citer à ce sujet le brevet français 77 04510 du 17/02/1977 (BASF) et les brevets US 5,504,247 (Sulzer) et 5,831,124 (BASF) et 6,482,981 (Nippon Shokubai) dont certains visent la purification de l'acide acrylique synthétisé par oxydation du propylène.

La technique la plus utilisée est la cristallisation fractionnée en film tombant, cristallisation dynamique, éventuellement associée à une cristallisation statique en milieu fondu.

La cristallisation à film tombant est généralement opérée dans un échangeur tubulaire, multitubulaire en pratique, chaque tube étant alimenté en continu (en tête), par :
- un flux liquide (solution ou fondu), du composé à purifier, l'acide acrylique (AA) dans le procédé, tombant en film de préférence le long de la paroi interne du tube, réceptionné en pied de tube et recyclé en tête (boucle fermée) pendant le temps nécessaire à la cristallisation de la quantité de composé (AA) décidée par l'opérateur,
- un flux de fluide caloporteur, par exemple éthylène glycol - eau ou méthanol -eau, tombant en film, de préférence le long de la paroi externe du tube, également recirculé tout au long de la cristallisation au sein du tube et qui apportera le froid ou la chaleur nécessaire à l'opération des étapes de chacun des étages.

Le procédé est une combinaison d'étages successifs, qui comprennent chacun 3 étapes :
- cristallisation : la température du fluide caloporteur est abaissée selon un gradient de température négatif à partir d'une température légèrement supérieure à la température de cristallisation de l'acide acrylique dans le milieu, de l'ordre de 14 °C. Des cristaux se forment en couche de plus en plus épaisse à la surface des tubes. Lorsque environ 30 à 80% d'AA mis en circulation est cristallisé, après égouttage, on transfert la fraction liquide restante (eaux-mères riches en impuretés) dans un récepteur.
- ressuage : la température du fluide caloporteur est réchauffée selon un gradient de température positif pour éliminer par fusion les impuretés emprisonnées sous forme d'inclusions dans la couche de cristaux d'acide acrylique en formation; celles-ci sont principalement localisées au niveau de la couche la plus externe qui est en contact avec le flux recirculé de plus en plus riche en impuretés. Lors du ressuage, les premières molécules à fondre sont des mélanges eutectiques d'impuretés et d'AA, les impuretés localisées au sein de la couche de cristaux migrent vers la couche externe, c'est-à-dire celle qui était en contact avec le flux recirculé. Une faible partie de cette couche de cristal est ainsi fondue et transférée dans un récepteur, de préférence le même récepteur que celui des eaux-mères récupérées lors de l'étape de cristallisation. A cette étape de ressuage, on peut substituer une technique de lavage qui consiste à éliminer les impuretés présentes à la surface par lavage avec de l'AA pur, introduit préférentiellement à température légèrement plus élevée que la température de fusion de la couche d'AA. Cette technique est cependant a priori moins efficace.
- fusion : la température du fluide caloporteur est rapidement augmentée au-delà du point de fusion de l'AA (14°C) et doit de préférence rester inférieure à une température maximum au delà de laquelle on peut craindre une polymérisation (explosive) du milieu : cette température maximum est de l'ordre de 35-40°C pour rester en sécurité pour faire fondre la couche de cristaux d'AA purifié. Le liquide purifié récupéré est placé dans un deuxième récepteur.

A partir du flux à purifier, l'ensemble des 3 étapes décrites représente un premier étage de purification. Le liquide purifié à l'issue de ce premier étage peut à nouveau subir une succession des 3 étapes décrites dans un 2ème étage de purification (phase de purification). Les eaux mères issues de ce 2ème étage sont plus pures que celles de l'étage précédent et peuvent donc être utilisées en mélange avec une nouvelle charge d'AA à purifier dans l'étage n°1. La même opération peut être réalisée dans un troisième étage de purification, les eaux mères de ce troisième étage pouvant être recyclées dans la charge du 2^{ème} étage, le produit pur étant récupéré par fusion des cristaux. D'une manière générale, les eaux-mères de l'étage de purification "n" peuvent être recyclées en les mélangeant avec le flux d'alimentation de l'étage de purification "n-1"

Lors des phases de purification, les inhibiteurs de polymérisation contenus dans les mélanges à purifier sont traités comme des impuretés et sont donc éliminés dans les eaux-mères. Pour éviter la formation de polymère dans le cristallisat fondu, on ajoute préférentiellement un inhibiteur compatible en nature et concentration avec l'usage final du monomère. Cet ajout sera en particulier mis en oeuvre lors de la dernière étape de fusion d'un étage alimenté par un flux exempt d'inhibiteur de polymérisation, comme par exemple le dernier étage de purification "n" alimenté uniquement avec un flux purifié de l'étage "n-1".

Les eaux-mères collectées à la suite du premier étage de purification peuvent être traitées dans un étage "-1" selon le même processus à trois étapes. Le cristallisat récupéré peut être utilisé comme complément de charge d'alimentation du premier étage. Les eaux mères de l'étage "-1" sont alors traitées selon le même processus pour une nouvelle séparation dont le cristallisat entrera comme charge de l'étage juste supérieur et les eaux mères soumises à nouveau au processus dans un étage inférieur "-2". Les étages "-1", "-2", etc... constituent les étages de concentration (les étages successifs permettent de concentrer les impuretés dans les flux d'eaux-mères). De manière générale, les eaux-mères des étages de concentration "n" sont traitées selon le même processus à 3 étapes dans l'étage postérieur "n-1". La répétition de ces opérations (phase de concentration) permettra de concentrer les impuretés dans un flux d'eaux mères de plus en plus riche en impuretés tandis que les fractions d'acide acrylique pur seront remontées vers l'étage initial. Ainsi on peut récupérer l'acide acrylique entraîné dans les eaux-mères initiales pour améliorer le rendement de récupération et par ailleurs obtenir un mélange « enrichi » en impuretés et en acide propionique.

Les étages de concentration successifs sont caractérisés par des flux d'eaux-mères de plus en plus concentrées en impuretés et en acide propionique, au fur et à mesure qu'on accumule ces étages. Ce faisant, la température de cristallisation de ces mélanges est de plus en plus basse, ce qui a pour effet d'augmenter le coût énergétique de refroidissement. Par ailleurs, le temps nécessaire pour cristalliser une même quantité d'acide acrylique est de plus en plus long, ce qui a pour conséquence de diminuer la productivité de la purification pour une même surface de cristallisation. Par conséquent, on arrêtera en général de préférence le nombre des étapes de concentration avant que la concentration totale des impuretés et en acide propionique dans les eaux-mères ne dépasse 50% poids du flux.

Selon la pureté du produit de départ, du produit purifié attendu et le rendement de récupération souhaité, le procédé complet pour une qualité initiale d'AA de type « technique » comprend préférentiellement de 1 à 5 étages de purification d'acide acrylique et de 1 à 5 étages pour la concentration des impuretés et de l'acide propionique, plus préférentiellement de 1 à 4 étages de purification et de 1 à 4 étages pour la concentration des impuretés et de l'acide propionique. Ceci est un avantage pour le procédé selon l'invention car ces étages de purification et de concentration nécessitent la consommation de beaucoup d'énergie ; un nombre limité d'étages permet d'obtenir un procédé plus économique tout en obtenant un bon rendement en acide propionique.

Pour améliorer encore le rendement de récupération, on peut aussi réaliser le dernier étage de concentration dans un cristalliseur statique. Dans ce cas, le mélange à cristalliser est placé en contact avec une paroi refroidie. Il peut s'agir par exemple d'un échangeur constitué de plaques métalliques circulées par un fluide caloporteur, plongées dans une cuve contenant les eaux-mères de cristallisation des étages précédents. L'AA forme une couche de cristal sur la paroi des plaques et on récupère les eaux-mères concentrées en acide propionique et en impuretés.

Lors du procédé selon l'invention, on isole au moins un flux d'eaux-mères, préférentiellement le flux d'eaux-mères du dernier étage de concentration.

Selon l'invention, on réalise l'hydrogénation du flux d'eaux-mères isolé lors de la cristallisation fractionnée en présence d'hydrogène moléculaire pour obtenir de l'acide propionique.

Le flux d'eaux-mères comprend préférentiellement de 50 à 90% en masse d'acide acrylique.

Cette hydrogénation peut être mise en oeuvre en phase liquide ou en phase gazeuse.

Par exemple, on peut réaliser l'hydrogénation :
- par catalyse homogène en phase liquide, le catalyseur pouvant être un complexe ruthenium-phosphine et le solvant étant le méthanol, à une température d'environ 60°C et à une pression d'environ 3MPa ;
- par catalyse hétérogène en phase gazeuse sur un catalyseur d'hydrogénation, par exemple cuivre-zinc déposé sur un oxyde d'aluminium, la réaction est alors effectuée en lit fixe à une température comprise entre 250°C et 350°C et à une pression comprise entre 1 atm et environ 6 atm ;
- par catalyse hétérogène sur un catalyseur de palladium appliqué sous forme d'une solution de sel de palladium liquide absorbée sur un support poreux tel que l'acide silicique ou un charbon actif, le sel étant par la suite réduit pour former le palladium métallique. Un avantage de ce procédé est qu'il peut être réalisé dans des conditions « douces », c'est-à-dire à des températures de 20 à 80°C et des pressions d'hydrogène de 1 à 10 atm, ce qui permet de limiter les réactions de polymérisation de l'acide acrylique.

Ce procédé est décrit en détail dans les pages 2 à 4 du document FR2219927, dont le contenu est intégré par référence.

On peut également citer les documents Chem. Prum., 37 (1987) p.651 à 653 et Electroanalytical Chemistry, 60 (1975) p.75 à 80 qui décrivent d'autres procédés d'hydrogénation d'acide acrylique en acide propionique.

Préférentiellement, l'hydrogénation est réalisée en phase gazeuse : selon cette variante, la catalyse d'hydrogénation est moins gênée par la présence éventuelle d'inhibiteurs de polymérisation.

En phase liquide, dans le cas où on a utilisé un inhibiteur de polymérisation soufré lors des étapes précédentes de séparation par distillation, on préfère réaliser, avant hydrogénation, une étape de purification préalable des eaux-mères, par exemple par distillation, en ajoutant éventuellement un inhibiteur de polymérisation non soufré lors de cette purification. On peut également utiliser une « masse de captation » avant hydrogénation, c'est à dire placer des composés solides capables de piéger les inhibiteurs soufrés, tels que le ZnO, TiₓCe_{y}O₂ tel que décrit dans la demande US 2009/065400, et/ou des métaux supportés tels que Mo et/ou Ni et/ou Co sous forme d'oxyde ou de sulfure avant d'introduire le catalyseur d'hydrogénation ou en amont de celui-ci.

La solution d'acide propionique issue de la réaction d'hydrogénation comprend des impuretés, comme l'acide acétique, facilement séparables par une étape supplémentaire de purification par distillation.

On obtient en fin de procédé une composition d'acide propionique bio-ressourcé ayant pour objet une composition d'acide propionique bio-ressourcé ayant une concentration en acide propionique supérieure à 85 % en poids, de préférence supérieure à 95 % en poids et de manière plus préférée supérieure à 99 % en poids.

L'invention porte aussi sur l'utilisation de ladite composition d'acide propionique bio-ressourcé ou de l'acide propionique bio-ressourcé obtenu selon le procédé de l'invention comme solvant, comme conservateur alimentaire ou pour la fabrication d'herbicide, pour la préparation d'acide perpropionique ou encore dans la préparation du propionate de vinyle qui est utilisé comme monomère dans des (co)polymères. L'application d'une étape de cristallisation fractionnée de l'acide acrylique combinée à l'hydrogénation des eaux-mères isolées à la fin de cette étape présente l'avantage de réaliser pleinement les objectifs recherchés dans la présente demande, c'est-à-dire obtenir un acide propionique bio-ressourcé et limiter les pertes de produit lors de la fabrication d'un acide acrylique purifié utilisant un procédé ex-glycérol.

Le procédé de fabrication de l'acide propionique selon l'invention est illustré par les exemples suivants.

### Exemple 1 : Fabrication d'acide acrylique brut à partir de glycérol

L'étape préliminaire consiste à purifier le glycérol brut obtenu à partir d'huile végétale, en éliminant les sels. La solution de glycérol brut est constituée en poids de 89,7% de glycérol, 3,9% d'eau, 5,1% de chlorure de sodium. Ce flux (6 400g) est envoyé en continu en alimentation d'un réacteur agité de 2 litres chauffé par un chauffe réacteur électrique extérieur. Les vapeurs de glycérol et d'eau sont condensées dans un réfrigérant et récupérées dans un récepteur. Cette opération de purification est réalisée sous pression de 670 Pa (5mm Hg). On obtient 5 710g d'une solution de glycérol exempte de chlorure de sodium.

Passant à l'étape (1) du procédé, on réalise la réaction de déshydratation du glycérol en acroléine et la condensation (1') d'une partie de l'eau. La réaction de déshydratation est effectuée en phase gaz dans un réacteur en lit fixe en présence d'un catalyseur solide constitué par une zircone tungstée ZrO₂-WO₃ à une température de 320°C, sous pression atmosphérique. Un mélange de glycérol (20% massique) et eau (80% massique) est envoyé dans un vaporiseur, en présence d'air, dans un ratio molaire O₂ / glycérol de 0,6 / 1. Le milieu gazeux sortant du vaporiseur à 290°C est introduit dans le réacteur, constitué d'un tube de diamètre 30mm chargé de 390ml de catalyseur, plongé dans un bain de sel (mélange eutectique KNO₃, NaNO₃, NaNO₂) maintenu à la température de 320°C.

En sortie du réacteur, le mélange réactionnel gazeux est envoyé en pied d'une colonne de condensation. Cette colonne est constituée d'une section inférieure remplie d'anneaux raschig, surmontée d'un condenseur circulé par un fluide caloporteur froid. La température de refroidissement dans les échangeurs est adaptée de façon à obtenir en tête de colonne une température des vapeurs de 72°C sous pression atmosphérique. Dans ces conditions, la perte d'acroléine en pied de colonne de condensation est inférieure à 5%.

Dans l'étape suivante (2), le mélange gazeux est introduit, après addition d'air (ratio molaire O₂ / acroléine de 0,8 / 1) et d'azote en quantité nécessaire pour obtenir une concentration d'acroléine de 6,5% molaire, en alimentation du réacteur d'oxydation de l'acroléine en acide acrylique. Ce réacteur d'oxydation est constitué d'un tube de diamètre 30mm chargé de 480ml d'un catalyseur commercial d'oxydation d'acroléine en acide acrylique à base d'oxydes mixtes d'aluminium, molybdène, silicium, vanadium et cuivre plongé dans un bain de sel identique à celui décrit ci-dessus , maintenu lui à une température de 345°C. Avant introduction sur le lit catalytique, le mélange gazeux est préchauffé dans un tube plongeant également dans le bain de sel.

En sortie du réacteur d'oxydation, le mélange gazeux est introduit en pied d'une colonne d'absorption, étape (3), fonctionnant sous pression atmosphérique. Cette colonne est remplie de garnissage vrac en inox du type ProPak. En partie inférieure, sur 1/3 de sa hauteur totale, la colonne est équipée d'une section de condensation, en tête de laquelle on recycle une partie du mélange condensé récupéré en pied de colonne, après refroidissement dans un échangeur externe. La partie supérieure de la colonne est refroidie par échange thermique à travers la paroi. La température des vapeurs en tête de colonne est de 25°C, celle de la solution aqueuse d'acide acrylique brut obtenue en pied de colonne est de 35°C. Le produit obtenu en pied (acide acrylique brut) contient 40% d'eau et un mélange d'acide acrylique (produit majoritaire) et d'impuretés, présentes dans des ratios massiques "impuretés / AA" indiqués dans le tableau 3 ci-dessous. On introduit en continu, dans la boucle de recirculation en pied de colonne, une solution aqueuse d'hydroquinone (HQ), à concentration de 0,1% poids par rapport à l'acide acrylique.

### Exemple 2 : purification de l'AA brut obtenu ex glycérol en AA technique

La solution aqueuse obtenue est soumise à une étape (4) de séchage par une distillation pour éliminer l'eau sous forme d'un mélange azéotropique avec la méthylisobutylcétone (MIBK). La colonne, garnie d'éléments ProPak représentant une efficacité de 15 plateaux théoriques, est alimentée en son milieu par l'AA brut, et en tête par la MIBK, dans un ratio massique MIBK / eau contenue dans l'AA brut de 3 / 1. Une solution de stabilisants dans la MIBK est injectée en continu en tête de colonne, contenant les stabilisants hydroquinone, phénothiazine et dibutyldithiocarbamate de butyle (respectivement : 35ppm, 70ppm, 35ppm par rapport à l'acide acrylique contenu dans le flux d'alimentation). Le mélange azéotropique distille à une température de tête de 45°C sous une pression de 1,2.10⁴ Pa.

L'acide acrylique déshydraté récupéré en pied de colonne ne contient plus que 0,4% d'eau.

Il est envoyé, étape (5), en alimentation d'une colonne d'étêtage, qui permet d'éliminer en tête, les composés légers, essentiellement l'acide acétique. Cette colonne, garnie d'éléments ProPak (20 plateaux théoriques), est alimentée en son milieu par le flux d'AA déshydraté, et on distille en tête un flux riche en acide acétique sous pression de 1,3.10⁴ Pa, à une température de tête de 77°C, avec un taux de reflux de 7 / 1. En tête de colonne de distillation, on introduit une solution de stabilisants dans l'acide acrylique technique contenant les stabilisants hydroquinone et dibutyldithiocarbamate de butyle (400 ppm par rapport à l'acide acrylique contenu dans le flux d'alimentation). Le rendement de récupération de l'acide acrylique dans cette étape est de 97%.

L'acide acrylique étêté récupéré en pied de cette colonne titre 0,07% d'acide acétique. Il est envoyé, étape (6) en alimentation d'une colonne d'équeutage garnie de 17 plateaux perforés à déversoirs, qui permet d'éliminer les composés lourds en pied. Cette colonne fonctionne sous une pression de 6,7.10³ Pa, avec une température de tête de 73°C, avec un taux de reflux de 0,5 / 1. Au niveau du plateau supérieur de la colonne de distillation, on introduit une solution de stabilisants dans l'acide acrylique technique contenant les stabilisants phénotiazine et dibutyldithiocarbamate de butyle (400ppm par rapport à l'acide acrylique contenu dans le flux d'alimentation) et le flux de distillat condensé est additionné d'une solution d'hydroquinone dans l'AA pur (200ppm par rapport à l'acide acrylique distillé). L'acide acrylique obtenu en tête de colonne constitue l'acide acrylique technique (AAT).

Les analyses de l'acide acrylique de grade technique montrent que le produit contient 0,07% d'acide acétique, 0,66% d'acide propionique, 0,11% d'anhydride maléique, 0,11% d'eau, 0,023% d'acide 2-butenoïque, 0,01 % de furfural, 0,02% de benzaldéhyde, 0,01 % de protoanémonine et 0,02% d'acroléine.

Le rendement de récupération de l'acide acrylique dans cette étape est de 95,5%.

### Exemple 3 : Fabrication d'acide propionique à partir d'acide acrylique technique ex glycérol purifié par cristallisation (1)

Le flux d'acide acrylique de qualité technique obtenu dans l'exemple 2 est soumis à une série d'étages de purification et de concentration par cristallisation fractionnée, telles que décrits dans la présente demande. Le montage utilisé est un cristalliseur à flux tombant constitué d'un tube vertical en inox rempli de fluide caloporteur (mélange éthylèneglycol - eau) circulant en circuit fermé, via une pompe, à travers un échangeur de chaleur externe programmable en rampe de température (bain cryostatique Lauda). Ce tube est alimenté en tête sous forme d'un film liquide coulant uniformément sur sa paroi externe. Le liquide constitué du mélange à cristalliser, récupéré dans un bac récepteur en pied, recircule en boucle dans un circuit calorifugé pour alimenter à nouveau le tube en tête, via une pompe.

Le flux d'acide acrylique technique est soumis à une série de plusieurs étages successifs de purification, chaque étage comprenant les étapes suivantes :
- cristallisation : le fluide caloporteur est refroidi rapidement de façon à abaisser la température du film tombant d'acide acrylique jusqu'à la température de cristallisation de l'acide acrylique dans le mélange, déterminée préalablement à partir d'un échantillon du mélange à purifier, puis un gradient négatif de température est imposé au fluide caloporteur, de 0,1 à 0,5 °C/min. Lorsque le volume d'acide acrylique cristallisé, mesuré par différence en évaluant le niveau de liquide dans le récipient collecteur en pied de cristalliseur, atteint 70% du mélange de départ, la recirculation du film tombant de mélange à purifier est stoppée, et le tube est égoutté. Le mélange liquide des eaux mères obtenues ainsi est séparé et stocké dans une recette.
- ressuage : le fluide caloporteur est réchauffé de façon à provoquer la fusion d'une partie (5%) de la couche d'acide acrylique cristallisée à la surface du tube. Les eaux de cette étape de ressuage sont collectées et stockées dans la même recette que les eaux mères de l'étape précédente
- fusion : le fluide caloporteur est rapidement réchauffé jusqu'à une température de 30°C jusqu'à fusion totale de la couche cristallisée. Le flux liquide purifié est placé dans une recette différente

Le produit purifié par fusion dans la dernière étape du premier étage de purification est envoyé au deuxième étage de purification, où il sera soumis à une nouvelle série des 3 étapes de purification dans les mêmes conditions opératoires. Les eaux -mères du deuxième étage de purification sont ensuite mélangées avec une nouvelle charge du flux d'alimentation d'AA technique dans l'étage 1. Ce processus est répété ainsi jusqu'à obtenir la qualité désirée dans le produit purifié fondu.

Pour limiter les pertes d'acide acrylique concentrées dans les eaux-mères de premier étage de purification, on réalise une série d'étages successifs de concentration, présentant les mêmes étapes que les étages de purification, dans lesquels le cristallisat de l'étage "n-1" est envoyé en alimentation de l'étage "n" et les eaux-mères de cet étage "n-1" sont envoyées en alimentation de l'étage "n-2".Ces étages sont réalisés dans les mêmes conditions opératoires que les étages de purification, hormis le volume d'acide acrylique cristallisé visé, avant de passer de l'étape de cristallisation à l'étape de ressuage, qui est de 60% du produit alimenté.

Le dernier étage de cristallisation est réalisé en mode statique. Le flux à purifier est placé dans un récipient en inox à double paroi circulé par un fluide froid maintenu à la température de cristallisation du milieu, déterminée auparavant par une mesure de température de cristallisation. Dans ce récipient, on immerge un tube vertical en inox rempli de fluide caloporteur (mélange éthylène glycol - eau) circulant en circuit fermé, via une pompe, à travers un échangeur de chaleur externe programmable en rampe de température.

Dans une première étape, on abaisse rapidement la température du fluide caloporteur dans le tube jusqu'à la température de cristallisation du milieu, puis on impose un gradient de température négatif de 0.1 à 0.5°C/min. Lorsque le volume cristallisé atteint environ 50% du produit de départ, on élimine les eaux mères, puis on procède à une étape de ressuage et enfin à l'étape de fusion, comme dans les étages supérieurs de cristallisation en mode dynamique.

Appliqué à l'acide acrylique technique obtenu à partir de glycérol au terme des étapes de purification de l'exemple 2, une succession de 4 étages de purification et 3 étages de concentration dont un étage de cristallisation en mode statique a permis d'obtenir de l'acide acrylique de qualité "glacial" contenant 50 ppm d'acide acétique, 410 ppm d'acide propionique, moins de 1ppm d'anhydride maléique, moins de 80 ppm d'eau, moins de 1ppm d'acide 2-butènoïque, moins de 1ppm de furfural, moins de 1 ppm de benzaldéhyde, moins de 1 ppm de protoanémonine, moins de 1 ppm d'acroléine.

Le rendement de récupération d'AA dans cette étape de purification est de 96,5%.

Les eaux-mères résiduelles de l'étage ultime de concentration ont la composition suivante :
Acide acrylique : 82,4 % en poids
Acide acétique : 1,7 % en poids
Acide propionique : 7,4 % en poids
Acide diacrylique : 0,6 % en poids
Furfurals : 0,3 % en poids
Benzaldéhyde : 0,6 % en poids
Eau : 2,5 % en poids
Hydroquinone : 0,5 % en poids

### Fabrication de la solution d'acide propionique

Un évaporateur tubulaire à double paroi en acier inoxydable (longueur du tube 100 cm, diamètre intérieur 2,5 cm, épaisseur de paroi 4 mm) a été garni sur toute sa longueur d'anneaux de Raschig en silice.

Un réacteur tubulaire à double paroi en acier inoxydable identique à l'évaporateur a été garni de bas en haut, d'abord sur une longueur de 5 cm, d'anneaux de Raschig, puis le réacteur tubulaire à double paroi a été garni d'un mélange homogène de 130 ml = 135,1 g du catalyseur d'hydrogénation Jonhson Mattey de type 50B (Pd à 0,3 % en poids sur gamma-A1203, en sphères de 2 mm) et de 226 ml d'anneaux de Raschig. Le reste de la longueur du réacteur tubulaire à double paroi n'a été garni que d'anneaux de Raschig Tant l'espace intermédiaire de l'évaporateur tubulaire à double paroi que celui du réacteur tubulaire à double paroi ont été garnis d'une huile formant un fluide caloporteur, qui présente une température de 185°C.

On a introduit (de haut en bas) dans l'évaporateur tubulaire à double paroi 10g/h des eaux-mères résiduelles. A contre-courant de ces eaux-mères, on a fait passer par l'évaporateur tubulaire 16 moles/h d'hydrogène moléculaire.

Le mélange d'acide acrylique et d'hydrogène moléculaire sortant de l'évaporateur a été immédiatement envoyé, de bas en haut, à travers le réacteur tubulaire à double paroi. L'extrémité de ce dernier est à la pression atmosphérique. La température au milieu du réacteur est d'environ 220°C. Dans un séparateur à 10°C, on a récupéré par condensation l'acide acrylique n'ayant pas réagi ainsi que l'acide propionique produit .

Le condensat contenait au bout d'une durée d'exploitation de 100h, 813 g d'acide propionique.

Après distillation, on récupère une solution d'acide propionique ayant une pureté de 99,1%.

### Exemple 4 : Fabrication d'acide propionique à partir d'acide acrylique technique ex glycérol purifié par cristallisation (2)

On effectue la même purification de l'AA technique ex glycérol par cristallisation que l'exemple précédent excepté qu'on réalise un étage de concentration supplémentaire en mode dynamique, soit 4 étages de purification et 4 étages de concentration, dont l'un en mode statique.

Le rendement de récupération d'AA dans cette étape de purification est de 99,3%.

Les eaux-mères récupérées après l'étape ultime de concentration ont la composition suivante :
54,4 % d'acide acrylique,
7,3 % d'eau,
8,9 % d'anhydride maléique,
4,4 % d'acide acétique,
16,7 % d'acide propionique,
et 1,5 % d'hydroquinone.

### Fabrication de la solution d'acide propionique

On réalise ici l'hydrogénation en phase liquide avec un catalyseur Pd/C.

Dans un autoclave on ajoute sous agitation magnétique à 60 °C, 200 g de solution des eaux mères, ainsi que 200 g d'acide propionique déjà récupéré, simulant un procédé avec recyclage, 50 g de catalyseur de Jonson Matthey de type 87G, puis on fait réagir le tout avec de l'hydrogène sous une pression absolue de 7 Bars pendant 2 heures. Après réaction, on récupère 340 g d'acide propionique.

### Exemple 5 : Fabrication d'acide propionique à partir d'acide acrylique étêté ex glycérol purifié par cristallisation

Les mêmes séries de traitement que l'exemple 3 (avec un étage de cristallisation statique) sont appliquées au flux obtenu en pied de la colonne d'étêtage (étape (5) de l'exemple 2).

Une succession de 4 étages de purification et 3 étages de concentration dont un étage de cristallisation statique a permis d'obtenir de l'acide acrylique de qualité "glacial" contenant moins de 50 ppm d'acide acétique, 500 ppm d'acide propionique, moins de 1 ppm d'anhydride maléique, moins de 100 ppm d'eau, moins de 1 ppm d'acide 2-butenoïque, moins de 1 ppm de furfural, moins de 1 ppm de benzaldéhyde, moins de 1 ppm de protoanémonine, moins de 1 ppm d'acroléine

Les eaux-mères résiduelles de l'étage ultime de concentration ont la composition suivante :
Acide acrylique : 67 % en poids
Acide acétique : 1,6 % en poids
Eau : 2,3 % en poids
Anhydride maléique : 9,4 % en poids
Acide propionique : 7,4 % en poids
Furfurals : 0,3 % en poids, et
Hydroquinone : 0,8 % en poids.

### Fabrication de la solution d'acide propionique

Un évaporateur tubulaire à double paroi en acier inoxydable (longueur du tube 85 cm, diamètre intérieur 3 cm, épaisseur de paroi 4 mm) a été garni sur toute sa longueur d'anneaux de Raschig (matériau SiO₂, verre quartzeux ; diamètre extérieur 3 mm, diamètre intérieur 2 mm, longueur 3 mm).

Un réacteur tubulaire à double paroi en acier inoxydable (longueur du tube 120 cm, diamètre intérieur 3 cm, épaisseur de paroi 4 mm) a été sur toute sa longueur garni d'un mélange homogène de 400 ml = 446 g du catalyseur d'hydrogénation de Johnson Matthey de type 48 (0,5 % en poids de Pd sur γ-Al₂O₃, extrudats en pastilles de 3 mm) et de 400 ml d'anneaux de Raschig. Tant l'espace intérmédiaire de l'évaporateur tubulaire à double paroi que celui du réacteur tubulaire à double paroi ont été garnis d'une huile formant caloporteur. L'huile formant caloporteur de l'évaporateur avait une température de 210°C, celle du réacteur avait une température de 180°C.

On a introduit (de haut en bas) dans l'évaporateur tubulaire à double paroi 10g/h des eaux-mères résiduelles. A contre-courant avec l'acide acrylique, on a fait passer à travers l'évaporateur tubulaire à double paroi 50 moles/h d'hydrogène moléculaire.

Le mélange d'acide acrylique et d'hydrogène moléculaire sortant de l'évaporateur a été immédiatement envoyé de bas en haut à travers le réacteur tubulaire à double paroi disposé au-dessus de l'évaporateur. L'extrémité du tube est à la pression atmosphérique. La température au milieu du réacteur est de 186°C. Dans un séparateur à 10°C, l'acide acrylique n'ayant pas réagi et contenu dans le courant de gaz produit ainsi que l'acide propionique formé sont séparés par condensation.

Après une durée d'exploitation de 20 h, on récupère dans le condensat 13,2 g d'acide propionique.

L'acide propionique produit selon l'invention est un acide bio-ressourcé fabriqué à partir de matières premières naturelles non fossiles.

## Revendications

1. Procédé de fabrication d'acide propionique bio-ressourcé à partir de glycérol comportant les étapes suivantes :
- déshydratation catalytique en phase gaz du glycérol en acroléine, (1)
- condensation partielle par refroidissement et extraction de l'eau contenue dans le milieu réactionnel de (1), (1')
- oxydation catalytique en phase gaz de l'acroléine en acide acrylique, (2)
- extraction de l'acide acrylique contenu dans l'effluent de l'oxydation par absorption avec un solvant, (3)
- séchage de la solution d'acide acrylique par distillation en présence d'un solvant non miscible à l'eau, (4)
- distillation de la solution ainsi obtenue pour éliminer les composés légers (étêtage), (5)
- distillation de la fraction lourde issue de l'étape précédente (5) pour éliminer les composés lourds (équeutage) (6)
combinées avec une étape d'extraction de l'acide acrylique par cristallisation fractionnée appliquée à l'un des effluents suivants : la fraction lourde de (4), fraction lourde de (5) ou fraction légère de (6) pour isoler des cristaux d'acide acrylique purifié et une solution d'eaux-mères appauvrie en acide acrylique
- hydrogénation catalytique des eaux mères isolées à l'étape de cristallisation fractionnée en présence d'hydrogène moléculaire pour former une solution d'acide propionique.
- séparation de l'acide propionique, de préférence par distillation.

2. Procédé selon la revendication 1 **caractérisé en ce que** la fraction liquide issue de (3) est soumise à une séparation (3') de l'acroléine résiduelle par distillation ou stripping avec un gaz.

3. Procédé selon l'une des revendications 1 et 2 **caractérisé en ce que** la fraction lourde de (4) est soumise à l'étape d'extraction de l'acide acrylique par cristallisation fractionnée.

4. Procédé selon l'une des revendications 1 et 2 **caractérisé en ce que** la fraction lourde de (5) est soumise à l'étape d'extraction de l'acide acrylique par cristallisation fractionnée.

5. Procédé selon l'une des revendications 1 et 2 **caractérisé en ce que** la fraction légère de (6) est soumise à l'étape d'extraction de l'acide acrylique par cristallisation fractionnée.

6. Procédé selon l'une des revendications précédentes dans lequel l'hydrogénation est réalisée en phase gazeuse.

7. Procédé selon l'une des revendications précédentes dans lequel on utilise un inhibiteur de polymérisation non soufré lors des étapes de distillation.

8. Procédé selon l'une des revendications précédentes dans lequel la cristallisation fractionnée comprend de 1 à 5 étages de purification d'acide acrylique et de 1 à 5 étages pour la concentration des impuretés et de l'acide propionique, plus préférentiellement de 1 à 4 étages de purification et de 1 à 4 étages pour la concentration des impuretés et de l'acide propionique.

## Patentansprüche

1. Verfahren zur Herstellung von aus biologischen Quellen stammender Propionsäure aus Glycerin, das folgende Schritte umfasst:
- katalytische Dehydratisierung von Glycerin zu Acrolein in der Gasphase, (1)
- partielle Kondensation durch Abkühlung und Extraktion des in dem Reaktionsmedium von (1) enthaltenen Wassers, (1')
- katalytische Oxidation von Acrolein zu Acrylsäure in der Gasphase, (2)
- Extraktion der in dem Austragsstrom der Oxidation enthaltenen Acrylsäure durch Absorption mit einem Lösungsmittel, (3)
- Trocknung der Acrylsäurelösung durch Destillation in Gegenwart eines nicht mit Wasser mischbaren Lösungsmittels, (4)
- Destillation der so erhaltenen Lösung zur Entfernung der leichten Verbindungen (Toppen), (5)
- Destillation der schweren Fraktion aus dem vorhergehenden Schritt (5) zur Entfernung der schweren Verbindungen (Tailing), (6)
kombiniert mit einem Schritt der Extraktion von Acrylsäure durch fraktionierte Kristallisation eines der folgenden Austragsströme: der schweren Fraktion von (4), der schweren Fraktion von (5) oder der leichten Fraktion von (6) zur Isolierung von Kristallen von gereinigter Acrylsäure und einer an Acrylsäure abgereicherten Mutterlaugenlösung
- katalytische Hydrierung der im Schritt der fraktionierten Kristallisation isolierten Mutterlaugen in Gegenwart von molekularem Wasserstoff zur Bildung einer Propionsäurelösung
- Abtrennung der Propionsäure, vorzugsweise durch Destillation.

2. Verfahren nach Anspruch 1, **dadurch gekennzeichnet, dass** die flüssige Fraktion aus (3) einer Abtrennung (3') des Restacroleins durch Destillation oder Strippen mit einem Gas unterworfen wird.

3. Verfahren nach einem der Ansprüche 1 und 2, **dadurch gekennzeichnet, dass** die schwere Fraktion von (4) dem Schritt der Extraktion der Acrylsäure durch fraktionierte Kristallisation unterworfen wird.

4. Verfahren nach einem der Ansprüche 1 und 2, **dadurch gekennzeichnet, dass** die schwere Fraktion von (5) dem Schritt der Extraktion der Acrylsäure durch fraktionierte Kristallisation unterworfen wird.

5. Verfahren nach einem der Ansprüche 1 und 2, **dadurch gekennzeichnet, dass** die leichte Fraktion von (6) dem Schritt der Extraktion der Acrylsäure durch fraktionierte Kristallisation unterworfen wird.

6. Verfahren nach einem der vorhergehenden Ansprüche, bei dem die Hydrierung in der Gasphase durchgeführt wird.

7. Verfahren nach einem der vorhergehenden Ansprüche, bei dem während der Destillationsschritte ein schwefelfreier Polymerisationsinhibitor verwendet wird.

8. Verfahren nach einem der vorhergehenden Ansprüche, bei dem die fraktionierte Kristallisation 1 bis 5 Acrylsäure-Reinigungsstufen und 1 bis 5 Stufen zum Aufkonzentrieren der Verunreinigungen und der Propionsäure, weiter bevorzugt 1 bis 4 Acrylsäure-Reinigungsstufen und 1 bis 4 Stufen zum Aufkonzentrieren der Verunreinigungen und der Propionsäure, umfasst.

## Claims

1. A process for the manufacture of bioresourced propionic acid from glycerol comprising the following stages:
- gas-phase catalytic dehydration of the glycerol to give acrolein, (I)
- partial condensation by cooling and extraction of the water present in the reaction medium from (1), (1')
- gas-phase catalytic oxidation of the acrolein to give acrylic acid, (2)
- extraction of the acrylic acid present in the stream from the oxidation by absorption with a solvent, (3)
- drying the acrylic acid solution by distillation in the presence of a water-immiscible solvent, (4)
- distillation of the solution thus obtained in order to remove the light compounds (topping), (5)
- distillation of the heavy fraction resulting from the preceding stage (5) in order to remove the heavy compounds (tailing), (6)
combined with a stage of extraction of the acrylic acid by fractional crystallization applied to one of the following streams: the heavy fraction from (4), heavy fraction from (5) or light fraction from (6), in order to isolate crystals of purified acrylic acid and a solution of mother liquors depleted in acrylic acid
- catalytic hydrogenation of the mother liquors isolated in the fractional crystallization stage in the presence of molecular hydrogen in order to form a propionic acid solution
- separation of the propionic acid, preferably by distillation.

2. The process as claimed in claim 1, **characterized in that** the liquid fraction resulting from (3) is subjected to a separation (3') of the residual acrolein by distillation or stripping with a gas.

3. The process as claimed in either of claims 1 and 2, **characterized in that** the heavy fraction from (4) is subjected to the stage of extraction of the acrylic acid by fractional crystallization.

4. The process as claimed in either of claims 1 and 2, **characterized in that** the heavy fraction from (5) is subjected to the stage of extraction of the acrylic acid by fractional crystallization.

5. The process as claimed in either of claims 1 and 2, **characterized in that** the light fraction from (6) is subjected to the stage of extraction of the acrylic acid by fractional crystallization.

6. The process as claimed in one of the preceding claims, in which the hydrogenation is carried out in the gas phase.

7. The process as claimed in one of the preceding claims, in which use is made of a sulfur-free polymerization inhibitor during the distillation stages.

8. The process as claimed in one of the preceding claims, in which the fractional crystallization comprises from 1 to 5 steps of purification of acrylic acid and from 1 to 5 steps for the concentration of the impurities and of the propionic acid, more preferably from 1 to 4 steps of purification and from 1 to 4 steps for the concentration of impurities and of the propionic acid.
